# EUROPEAN PATENT APPLICATION

(11) **EP 2 374 489 A2**
(43) Date of publication of application: **12.10.2011**
(21) Application number: 11151575.5
(22) Date of filing: 20.01.2011
(51) Int. Cl.: A61M 15/02

(54) **Electrohydrodynamic aerosolization device having a time varying voltage**

(30) Priority: 21.01.2010 US 336399 P
(71) Applicant: Battelle Memorial Institute, Columbus, OH 43201-2696 (US)
(72) Inventor: Triplett, II Michael D., New Albany OH 43054 (US); Lipp, Brian A., Columbus, OH 43235 (US)
(74) Representative: Clark, Jane Anne

(57) **Abstract**

The invention is directed to an improved electrohydrodynamic (EHD) apparatus having a time varying voltage component. The invention further relates to the use of such enhanced EHD apparatus to produce respirable and non-respirable aerosols from highly aqueous liquids, as well as the use of such aerosols. The combination of modifying surface rheology and superimposing a time varying waveform onto the direct current (DC) electrical field enables electrohydrodynamic aerosolization of high aqueous content formulations (>50% water) beyond what can be achieved through other means, including the individual gains by applying surface rheology modification and superimposing a sinusoidal waveform onto the DC electrical field individually.

## Description

### CROSS REFERENCE TO RELATED APPLICATION

This application claims the benefit of U.S. Provisional Patent Application Serial No. 61/336,399, filed January 21, 2010, entitled "ELECTROHYDRODYNAMIC AEROSOLATION DEVICE HAVING A TIME VARYING VOLTAGE", the entire disclosure of which is hereby incorporated by reference herein.

### BACKGROUND OF THE INVENTION

The invention is directed to an improved electrohydrodynamic (EHD) apparatus having a time varying voltage component. The invention further relates to the use of such enhanced EHD apparatus to produce respirable and non-respirable aerosols from highly aqueous liquids, as well as the use of such aerosols.

Devices and methods for forming fine sprays by particular EHD techniques are known. For example, U.S. Patent No. 4,962,885 to Coffee, incorporated by reference herein, describes a process and apparatus to form a fine spray of electrostatically charged droplets. More specifically, the process and apparatus comprise a conductive nozzle charged to a potential on the order of 1-20,000 volts, closely adjacent to a grounded electrode. A corresponding electric field produced between the nozzle and the grounded electrode is sufficiently intense to atomize liquid delivered to the nozzle, and thereby produce a supply of fine charged liquid droplets. However, the field is not so intense as to cause corona discharge, resulting in high current consumption. Uses of such liquid dispenser processes and apparatuses include sprayers for paint and spraying of crops.

More recently, there has been recognition that EHD spraying (aerosolization) devices are useful for producing and delivering aerosols of therapeutic active agents for inhalation by patients. In one particular example, described in U.S. Patent No. 6,302,331 to Dvorsky et al. incorporated by reference herein, fluid is delivered to a nozzle that is maintained at high electric potential relative to a proximate electrode to cause aerosolization of the fluid with the fluid emerging from the nozzle in a conical shape called a Taylor cone. One type of nozzle used in such devices is a capillary tube that is capable of conducting electricity. An electric potential is placed on the capillary tube which charges the fluid contents such that the fluid emerges from the tip or end of the capillary tube in the form of a Taylor cone. The Taylor cone shape of the fluid before it is dispensed results from a balance of the forces of electric charge on the fluid and the fluid's own surface tension. Due to the finite conductivity of most liquids, a thin liquid (on the order of 1 µm diameter) jet (with speeds up to 10m/s) emerges from the cone tip. Due to Rayleigh instability, the jet breaks up into a stream of mono-dispersed charged particles. The charge then causes the droplet stream to diverge into a conical aerosol spray. The Dynamics of a Steady Taylor cone electrospray Martin Bell (Ohio University, Athens, OH 45701), Maarten A. Rutgers (The Ohio State University, Columbus, OH 43210*) ;* Session LJ - Surface Tension Effects I. ORAL session, Tuesday morning, November 24, Jefferson, Adam's Mark Hote*l.* The resulting aerosol spray may remain charged or can be discharged to produce a neutral spay. Studies have shown that this aerosol (often described as a soft cloud) has a uniform droplet size and a high velocity leaving the tip but that it quickly decelerates to a very low velocity a short distance beyond the tip.

EHD sprayers produce charged droplets at the tip of the nozzle. Depending on the use, these charged droplets can be partially or fully neutralized (with a reference or discharge electrode in the sprayer device). The typical applications for an electrostatic sprayer, without means for discharging or means for partially discharging an aerosol would include a paint sprayer or insecticide sprayer. These types of sprayers may be preferred since the aerosol would have a residual electric charge as it leaves the sprayer so that the droplets would be attracted to and tightly adhere to the surface being coated. However, in other cases it may be preferred that the aerosol be completely electrically neutralized. For example, in the delivery of some therapeutic aerosols electric neutralization or discharge allows the aerosol to impact deep in the lung rather than adhere to the linings of the mouth and throat.

At the present time, inhalation therapy is a rapidly evolving technology. Numerous active drugs are being developed with the expectation that effective delivery of and treatment with these drugs will be possible by means of inhaled aerosols. Aerosolizing active ingredients requires a liquid composition with certain characteristics and properties that make the liquid composition compatible with the aerosolization process. The process of formulating particular active ingredients with the appropriate highly aqueous liquid carrier can be particularly challenging. Therefore, there is a need for basic or general aqueous liquid compositions which are compatible with a variety of active ingredients.

U.S. Pat. No. 4,829,996 to Noakes et al., U.S. Pat. No. 5,707,352 to Sekins et al. and U.S. Pat No. 6,503,481 to Browning et al., each of which is incorporated by reference herein, all disclose formulations suitable for use with electrostatic aerosol devices; however, despite this prior art, spraying highly conductive, highly aqueous formulations (solutions where the conductivity is around or greater than 12.5 µS/cm) remains challenging in the cone-jet mode at relatively high volumetric flow rates and low voltage requirements. Typically, spraying highly conductive , highly aqueous liquid formulations yields large particles, multi-modal distributions, and numerous discharge streamers. This undesirable outcome results from an imbalance between the electrical and physiochemical forces within the Taylor cone. While it is possible to aerosolize highly conductive, highly aqueous formulations without altering surface rheology by reducing the fluid's volumetric flow rate well below what would be practical for many applications including pulmonary therapeutics, it would be highly desirable to use an EHD device to aerosolize highly conductive, highly aqueous liquid formulations at high flow rates and at relatively high conductivities.

Hartman et al. [J. Aerosol Sci. Vol. 30, No. 7, pp. 823-849, 1999] discusses a force balance on an idealized Taylor cone under laminar flow conditions. Within the Taylor cone, the electrical forces of normal electric stress, tangential electric stress, and electric polarization stress resulting from the electric charge are balanced against the physiochemical forces of surface tension, viscosity, and gravity. As liquid conductivity increases (i.e. resistivity decreases), the electrical forces increase while the physiochemical forces, with surface tension being the most important, remain constant. Thus, the resulting force imbalance induces Taylor cone instability, leading to poor spraying. The force imbalance can lead to complex chaotic flow behavior [Marginean, I., Nemes, P. and Vertes, A., Order-Chaos-Order Transitions in Electrosprays: The Electrified Dripping Faucet, Physical Review Letters 11 August 2006, PRL 97, 064502 (2006)].

### SUMMARY OF THE INVENTION

Embodiments of the invention are directed to an improved electrohydrodynamic (EHD) apparatus having a time varying voltage component. Embodiments of this invention further relate to methods of using aerosols produced using the improved EHD device where such aerosols are produced from the highly conductive, highly aqueous liquid carrier vehicles. The inventors have discovered that by controlling critical surface viscoelastic properties, one is able to increase the efficiency (as measured by the combination of flow rate and applied voltage) of spraying highly aqueous, highly conductive formulations using the enhanced EHD aerosolization device. The inventors also have discovered that the combination of modifying surface rheology and superimposing a sinusoidal waveform onto the direct current (DC) electrical field within the EHD device enables electrohydrodynamic aerosolization of high aqueous content formulations (>50% water) beyond what can be achieved through other means, including the individual gains by applying surface rheology modification and superimposing a sinusoidal waveform onto the DC electrical field individually. It is unanticipated that the combination of surface rheology modification and superimposing alternating current would produce a benefit greater than the sum of the individual benefits

Embodiments of the present invention contemplate an aerosolization system that includes an aerosol generating device for aerosolization of a liquid that is connected to a voltage supply that is operative to generate a voltage including a high voltage DC component and a time-varying component. Embodiments of the present invention further contemplate that the liquid being aerosolized is a highly aqueous, highly conductive carrier liquid, where the carrier liquid containing an active ingredient (formulation) is capable of being aerosolized into small uniform particles. According to some embodiments, a predetermined quantity of a desired active ingredient can be delivered to a site of choice, e.g., an active pharmaceutical agent to the lungs of the user, with an electrostatic or electrohydrodynamic aerosol generating device. Critical properties of the formulation are provided for desirable, and preferably optimal, use of the formulation with such aerosol generating devices.

It may be appreciated that embodiments of the present invention provide formulations (compositions) of highly conductive, highly aqueous liquids which have certain preferred characteristics. These preferred characteristics cause aerosols generated from the compositions to also have particular preferred characteristics. A typical embodiment of this invention includes a liquid composition having predetermined surface rheological properties which facilitate aerosolization of the composition with an EHD aerosolization device.

Various objects and advantages of this invention will become apparent to those skilled in the art from the following detailed description of the preferred embodiment, when read in light of the accompanying drawings.

### BRIEF DESCRIPTION OF THE DRAWINGS

**Fig. 1** is a schematic diagram of an aerosolization system that is in accordance with the present invention.
**Fig. 2** is a circuit diagram for a high voltage source that is included in the system shown in **Fig. 1**.
**Fig. 3** is a schematic diagram of an alternate embodiment of the system that is shown in **Fig. 1**.
**Fig. 4** illustrates voltages utilized within the system shown in **Fig. 1**.
**Fig. 5** is a sectional view of an electrohydrodynamic (EHD) sprayer utilized in the system shown in **Fig. 1**.
**Fig. 6** is a sectional view of a nozzle that is included in the EHD sprayer shown in **Fig. 5**.
**Fig. 7** is a simplified representation of a pendent bubble generated by the EHD nozzle shown in **Fig. 5****.**

### DETAILED DESCRIPTION OF THE PREFERRED EMBODIMENT

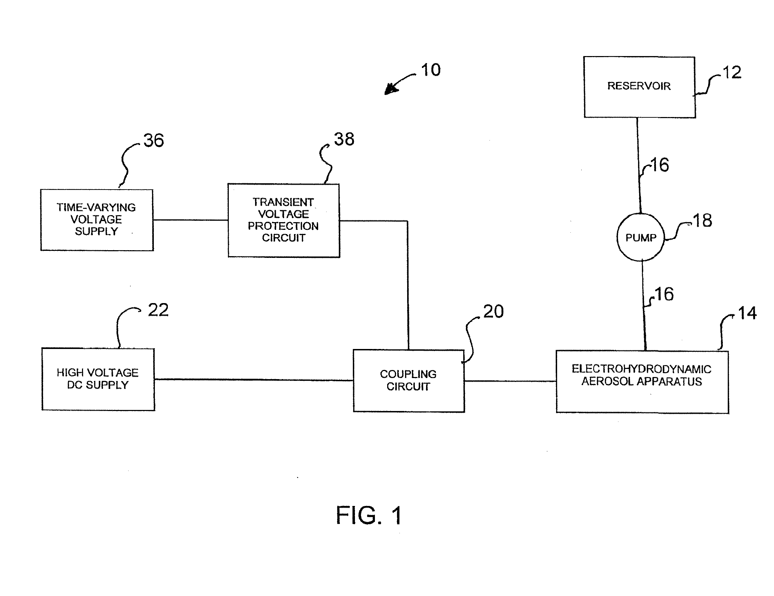

Referring now to the drawings, there is illustrated in **Fig. 1** a schematic diagram of an aerosolization system 10 that is in accordance with the present invention. The system 10 includes a reservoir 12 containing a fluid that is to be subjected to electrohydrodynamic aerosolization. The reservoir 12 supplies the fluid an aerosol apparatus 14 that converts the fluid into a cloud of droplets. As shown in **Fig. 1**, the fluid is conveyed to the aerosol apparatus 14 through a conduit 16 and pressurized by a pump 18. It will be appreciated that the inclusion of the pump 18 is meant to be exemplary and that that embodiments of the invention also may be practiced without a pump, such as, for example, when the fluid is gravity fed to the aerosol apparatus 14 (not shown) or the fluid may is drawn to the aerosol apparatus 14 by capillary action within the apparatus. The aerosol apparatus 14 is connected through a coupling circuit 20 to a high voltage Direct Current (DC) power supply 22.

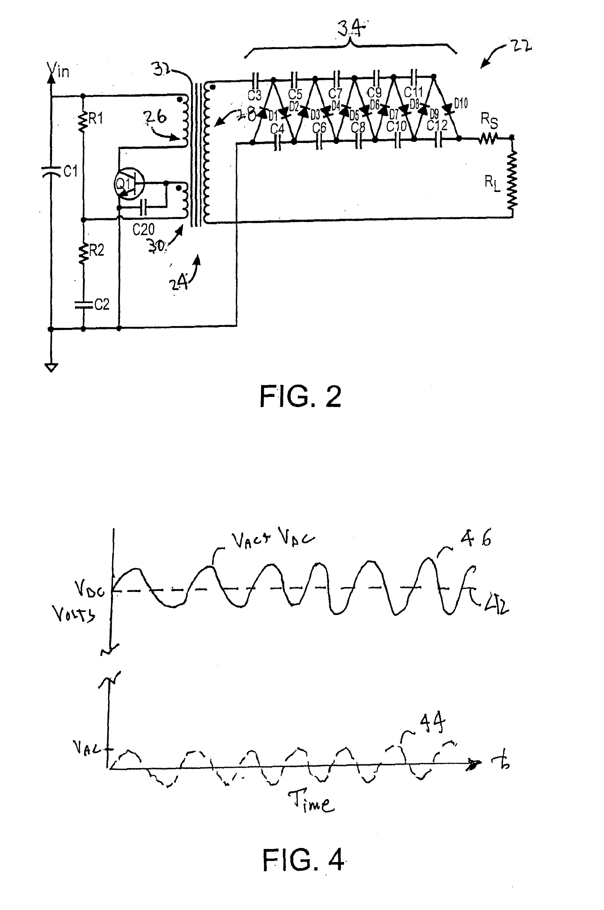

A typical high voltage DC power supply 22 that may be utilized is illustrated by the circuit diagram shown in **Fig. 2**. The high voltage DC power supply 22 includes a flyback transformer 24 having primary and secondary windings 26 and 28, respectively, with the secondary winding having more turns than the primary winding. The flyback transformer also includes a feedback winding 30. All three windings 26, 28 and 30 are wound upon a common core 32. The high voltage DC power supply 22 also includes a switching transistor Q1 that has a collector terminal connected to one end of the primary winding 26 and an emitter terminal connected to ground. The switching transistor Q1 has a base terminal connected through the feedback winding 30 to the common connection of first and second feedback winding bias resistors R1 and R2, respectively. The non-common connection end of the first resistor R1 is connected to a DC power supply Vᵢₙ while the non-common connection end of the second resistor R2 is connected through a tuning capacitor C2 to ground. The tuning capacitor C2 co-operates with the resistors R1 and R2 in the bias voltage divider to provide a time constant that determines the oscillation frequency of the input voltage to the flyback transformer. A large filter capacitor C1 is connected between the power supply Vᵢₙ and ground across the series connection of R1, R2 and C2. A compensation capacitor C20 is connected between the base and emitter terminals of the switching transistor Q1. Because the switching transistor emitter terminal is connected to ground, the compensation capacitor C20 is also connected between one end of the feedback winding 18 and ground.

The high voltage DC power supply 22 also includes a conventional Cockcroft-Walton voltage multiplier circuit 34 connected across the secondary winding 28 of the flyback transformer 24. The voltage multiplier circuit 34 includes a cascaded series of capacitors and diodes. During operation, the capacitors are cascade charged with each set of two capacitors and two diodes doubling the applied voltage at the output of the secondary winding 28. The output is then the sum of all of the voltages on the individual capacitors. The diodes control the current path through the capacitors to provide a constant output voltage Vₒᵤₜ that has little or no ripple. For the five sets of capacitors and diodes shown in **Fig. 2**, the voltage applied to the input of the voltage multiplier circuit 34 is doubled five times for a total of 10 times for the complete multiplier circuit. As an example of the operation of the high voltage DC power supply 22, an input voltage V_{IN} of four volts may generate a secondary winding voltage of 2 Kv which is then multiplied by ten to produce an output voltage V_{OUT} of 20 Kv.

While the multiplier circuit 34 shown in **Fig. 2** includes ten stages, it will be appreciated that embodiments of the invention also may be practiced with more or less stages than are shown in order to increase or decrease, respectively, the output voltage produced. The final stage of the multiplier circuit 34 is connected to an output resistor R_{S} that limits the output current as a protection for the users; however, the output resistor may be omitted. The resistor R_{L} connected between the output resistor R_{S} and the secondary winding 28 represents one of the inputs to the coupling circuit 20.

The high voltage DC power supply 22 is more fully described in US Patent Application No. 12/306,100, also PCT/US2007/01481, both of which are incorporated herein by reference. It will be appreciated that the power supply shown in **Fig. 2** is meant to be exemplary and that embodiments of the invention also may be practiced with other high voltage DC power supplies than the one shown in **Fig. 2**. Several such alternate high voltage DC power supplies are illustrated in the above referenced US Patent Application No. 12/306,100; however, the invention is not intended to be limited to only the supplies shown in the application.

Embodiments of the present invention contemplate that a time varying voltage is superimposed upon the output of the high voltage DC power supply 22. Accordingly, as shown in **Fig. 1**, the aerosolization system 10 also includes a time-varying voltage supply 36 that is connected through a transient voltage protection circuit 38 to the output of the high voltage DC power supply 22 by the coupling circuit 20. The output of the coupling circuit 20, which is the combination of the DC voltage from the DC power supply 22and the time-varying voltage from the time-varying voltage supply 36 is connected to the aerosol apparatus 14. The transient voltage protection circuit 38 functions as a buffer to protect the time-varying voltage supply 36 from transients when the high voltage DC power supply 22 is switched on or off and from any other spikes or ripples that may be generated by the DC supply.

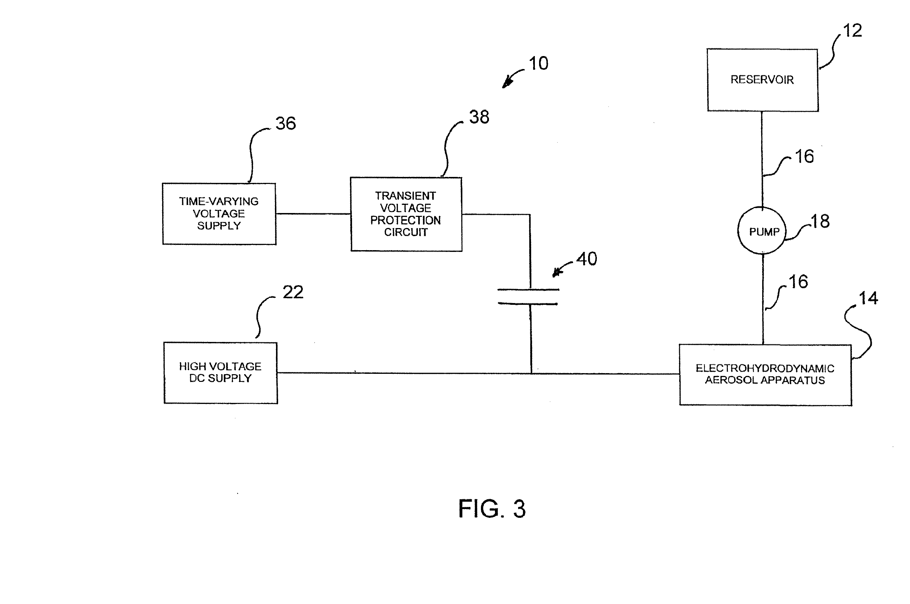

One method for implementing the coupling circuit 20 is illustrated in **Fig. 3** where components that are same as those shown in **Fig. 1** have the same numerical identifiers. As shown in **Fig. 3**, the coupling circuit 20 has been replaced by a coupling capacitor 40 connected between the output of the transient voltage protection circuit 38 and the output of the high voltage DC power supply 22. While a coupling capacitor 40 is shown in **Fig. 3**, it will be appreciated that other conventional coupling devices or circuits also may be utilized to practice the invention. For example, the output of the time-varying voltage supply 36 could also be magnetically coupled to the output of the high voltage DC power supply 22 (not shown).

The inventors contemplate that any time varying voltage may be added to the output of the high voltage DC power supply 22, such as, for example, an alternating voltage supply may be utilized for the time-varying voltage supply 36. For such a case, a sinusoidal voltage is superimposed upon the high voltage DC power supply output as illustrated in **Fig. 4** to produce the voltage applied to the aerosol apparatus 14. In **Fig. 4**, the horizontal dashed line labeled 42, representing the DC voltage, and having a magnitude of V_{DC}, is combined with the sinusoidal dashed line labeled 44, representing the AC voltage, and having a magnitude of V_{AC}, to produce the solid line labeled 46, representing the composite voltage that is the sum of V_{DC} and V_{AC}. The sum 46 is the voltage that is applied to the aerosol apparatus 14. It will be noted that, for clarity, the AC voltage 44 shown in **Fig. 4** has a magnitude that is less than that of the DC voltage 32; however, the **Fig. 4** is not to scale. The vertical axis in **Fig. 4** is shown as being discontinuous to emphasize this point. Additionally, it is contemplated that the frequency of the AC voltage may be varied. The inventors have successfully used AC voltages having a range of 2.0 volts to 4.6 volts and frequencies having a range of 75 kHz to 110 kHz. In comparison, typical DC voltage levels produced by the DC high voltage supply 20 would be in the range of 3 Kv to 30 Kv. However, it will be appreciated that the invention also may be practiced with voltages and frequencies that are not included in these ranges.

The invention contemplates a first embodiment, in which the voltage levels of the AC and DC supplies 36 and 22 and the frequency of the AC supply may be varied (not shown) and a second embodiment in which the voltage levels and the AC supply frequency are fixed. The first embodiment would be utilized with different fluids with the voltage levels and AC supply frequency adjusted to provide optimal performance for the specific fluid being aerosolized. In a similar manner, the second embodiment would be utilized when the same fluid is to be aerosolized and the voltage supply levels and AC supply frequency would be preset for those levels. It is further contemplated that a microprocessor or similar device may be included in the first embodiment (not shown) and programmed to adjust the voltage levels and AC frequency for different fluids. The user would merely enter a code representing the specific fluid through an interface (not shown) to set up the system for the specific fluid being utilized.

Aerosols having uniformly-sized particles and uniform distribution patterns are desirable over aerosols that do not possess these characteristics because they exhibit more desirable deposition properties such as for inhalation into the pulmonary tract of the user (i.e., where they have a higher respirable fraction). When used with compatible compositions, EHD aerosol generating devices can be adjusted to create substantially mono-modal aerosols having particles more uniform in size than aerosols generated by other devices or methods. Accordingly, one embodiment of the invention utilizes an EHD spray nozzle for the aerosol apparatus 14 shown in **Fig. 1**.

A typical EHD spray nozzle has a first electrode highly charged by connection to the high voltage supply and a second electrode that is grounded. For example, the nozzle may have a discharge electrode that could be at + or -10 kV while the nozzle is grounded. Thus, the nozzle is maintained at a high electric potential. Most EHD devices create aerosols by causing a liquid to form droplets that enter a region of high electric field strength. The electric field then imparts a net electric charge to these droplets, and this net electric charge tends to remain on the surface of the droplet. The repelling force of the charge on the surface of the droplet balances against the surface tension of the liquid in the droplet, thereby causing the droplet to form a cone-like structure known as a Taylor Cone. In the tip of this cone-like structure, the electric tangential and normal forces exerted on the surface of the droplet overcome the surface tension of the liquid, thereby generating a stream of liquid that disperses into many smaller droplets of roughly the same size. These smaller droplets form a mist which constitutes the aerosol cloud that the user ultimately inhales.

One type of nozzle used in EHD devices is a capillary tube that is capable of conducting electricity. An electric potential is placed on the capillary tube which charges the fluid contents such that as the fluid emerges from the tip or end of the capillary tube a so-called Taylor cone is formed. This cone shape results from a balance of the forces of electric charge on the fluid and the surface tension of the fluid. Desirably, the charge on the fluid overcomes the surface tension and at the tip of the Taylor cone, a thin jet of fluid forms and subsequently and rapidly separates a short distance beyond the tip into an aerosol. Studies have shown that this aerosol (often described as a soft cloud) has a fairly uniform droplet size and a high velocity leaving the tip but that it quickly decelerates to a very low velocity a short distance beyond the tip.

EHD sprayers produce charged droplets at the tip of the nozzle. Depending on the use, these charged droplets can be neutralized (with a reference or discharge electrode in the sprayer device) or the droplets may be left charged. The typical applications for an EHD sprayer without reference or discharge electrodes would be a paint sprayer or insecticide sprayer. Charged droplets in these types of sprayers may be preferred since the aerosol would be attracted to and tightly adhered to the surface being coated. However, with EHD apparatus used to deliver therapeutic aerosols, it is preferred that the aerosol be completely electrically neutralized prior to inhalation by the user to permit the aerosol to reach the pulmonary areas where the particular therapeutic formulation is most effective. Other drug delivery applications may dictate a small residual charge on the aerosol to accomplish some particular therapy.

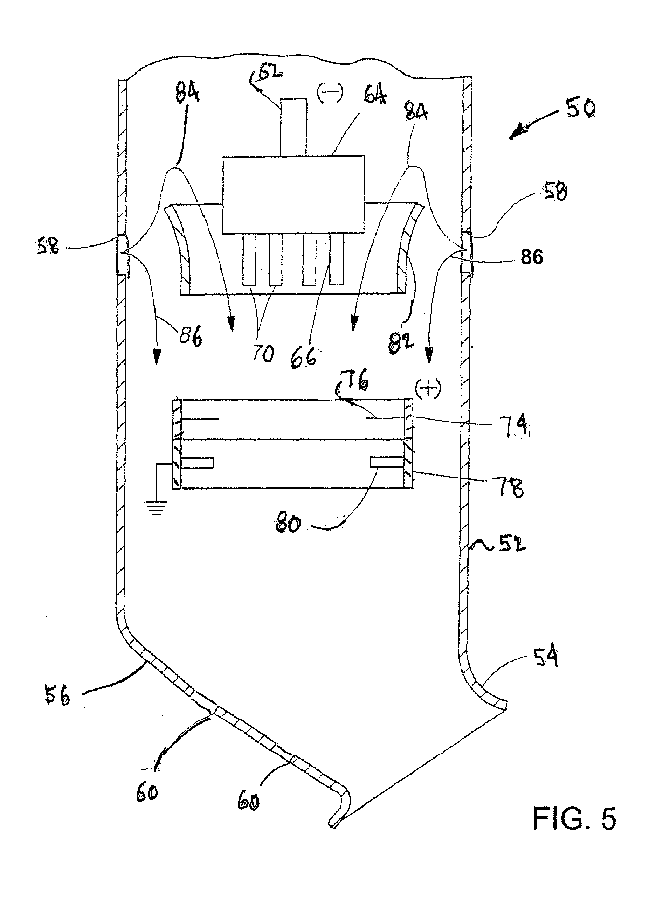

Referring again to the drawings, there is illustrated in **Fig. 5** a typical EHD sprayer 50. The sprayer 50 could, for example, be used in a hand held EHD device for pulmonary drug delivery. A housing 52 includes an exit opening 54. For pulmonary drug delivery, exit 54 could, for example, directly contact a user's mouth or connect to a face mask or other interface leading to the user's mouth. As shown, slanting wall or elbow 56 is used to change the direction of an aerosol from essentially vertical to more horizontal for ease of delivery to an upright user. The housing 52 may contain optional air holes 58 and/or 60 for entry of any air needed during an inhalation cycle, for example. Clearly, the higher the ratio of air through optional holes 58 to that through optional holes 60, the greater the flow past the spray nozzles. Alternatively, a source of air or other gas could be provided within the device in the region near the air holes upstream of the location where the aerosol is produced. The source of gas could, for example, be from a pressurized container, a mechanical device such as a bellows or other common source. The wall 56 may contain optional air holes for additional air flow and to keep droplets off the wall.

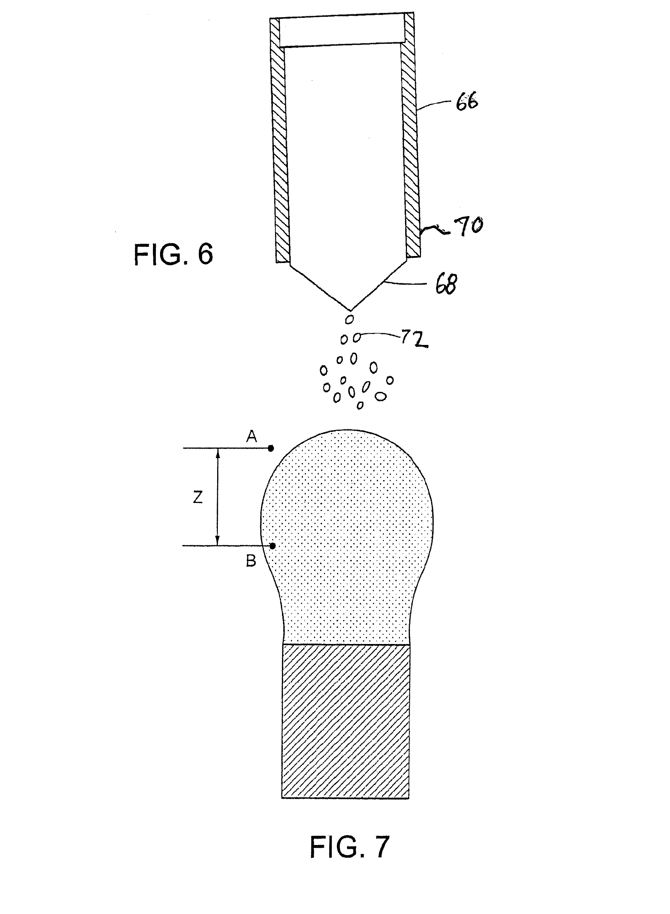

The sprayer 50 includes fluid delivery means that include a connector 62 to the reservoir 12 containing the fluid to be aerosolized and an optional manifold 64. The fluid is delivered via the connector 62 and the manifold 64 to spray nozzles 66. The spray nozzles 66 may be any means for delivery of the fluid for producing a Taylor cone 68 at a spray end or tip 70 of the nozzle, as shown in the sectional view of one of the nozzles in **Fig. 6** and as is well known within the EHD spraying art. The composite voltage delivered by the coupling circuit 20 is applied to the spray nozzles 66 to produce a high electric field around the spray tip. In **Fig. 5**, the charge on the spray nozzle is shown as negative, but a positive charge could also be used. When the charge on the spray nozzle is high enough, the surface tension of the fluid is exceeded and an aerosol 72 is produced.

Downstream of the spray nozzles is a first ring 74 having one or more discharge electrodes 76. In some applications favoring neutral droplets, the charge on the droplets is discharged to a chosen degree by means of the discharge electrode having an electrical charge the opposite polarity of the droplets. In a preferred mode, the droplets have a negative charge and the discharge electrode produces positive ions from the gas molecules near an ionization site. Ions are favorably produced near sharp points or edges of the discharge electrodes. The discharge electrodes are optional and may not be used when no discharge of the aerosol is desired. Some applications may require a partial discharge in which case, the position and the charge on the discharge electrode may be customized to provide partial discharge.

Downstream of the discharge electrodes 76 is an optional second ring 78 and one or more optional reference electrodes 80. When the discharge electrodes 76 are at a positive potential, the reference electrodes 80 are at a potential that is negative with respect to that of the discharge electrodes (which potential may preferably be ground potential). It is preferable to avoid having any positive ions migrate to the negative spray tip where they may interfere with the electric field around the Taylor cone.

A gas deflector 82 is shown as an annular frustoconical member completely around the spray nozzles 66. The gas or air deflector 82 may be of any shape that will assist in moving a gas along the desired path. Its purpose is to promote a first portion of the airflow 84 past the nozzles 66, the spray tips 70 and the Taylor cones 68 and to deflect a second portion of the airflow 86 away from the spray tips to later merge with the first portion downstream of the spray tips. Gas or air deflector 82 may have shaped walls to accomplish this. As shown in FIG. 5, the deflector also serves to direct a portion of the airflow 84 entering through holes 58 (or from another source) down over the spray tips.

An embodiment of the apparatus as illustrated by the drawings is capable of aerosolization of any liquid containing an active ingredient or active agent; however, the apparatus is especially useful in the production of respirable and non-respirable aerosol particles where the carrier liquid for the active agent is "highly aqueous" and also "highly conductive."

The enhanced EHD apparatus is useful to spray any liquid formulations comprising a carrier vehicle in which an active agent is dissolved or suspended. As described in detail below, the enhanced EHD apparatus is particularly useful in a method of aerosolization of highly aqueous, highly conductive liquid formulations.

As used herein, the term "highly aqueous" refers to a liquid carrier vehicle which is composed of at least 50 percent water and preferably at least about 70 percent water (v/w%).

The term "highly conductive" is used herein to refer to a highly aqueous "formulation" which has the following surface properties: a surface viscoelastic modulus of from about 0.5 mN/m to about 10 mN/m, a phase angle of from about 0.5 degrees to about 90 degrees, and a conductivity of from about 5.0 µSiemens/cm to about 1000 µSiemens/cm and preferably from about 12.5 µSiemens/cm to about 400 µSiemens/cm.

For electrostatic spraying of highly aqueous, highly conductive formulations it is preferred that the aqueous solution (or suspension) has both a low overall surface viscoelastic modulus (E) and high phase angle(δ) at short oscillation periods, particularly at a 1s oscillation period, to facilitate aerosolization of highly aqueous, highly conductive formulations. In practical terms, the surface becomes more viscous as E decreases and phase angle (δ) increases. As E decreases, the surface tension increases less during drop expansion. As phase angle (δ) increases, surface tension increases are dampened more effectively. In a preferred embodiment, E should be less than 10 mN/m and more preferably less than 7.5 mN/m, while phase angle (δ) should preferably be more than 10 degrees and more preferably more than 20 degrees.

Judicious selection of the surfactant(s) used in the highly aqueous liquid formulations described herein, as well as the selection of the co-solvent will enable one to prepare highly conductive, highly aqueous formulations which can be efficaciously aerosolized using the EHD device described in detail above and as illustrated in **Fig. 1** and **Fig. 2**.

The term "formulation" is used herein to mean an active agent dissolved or suspended in the highly aqueous, highly-conductive" carrier liquid described herein. Depending on whether the resulting aerosol is respirable or non-respirable, the active agent will be a drug (respirable) or an agricultural active agent e.g., herbicide, insecticide, or a biologically active agent for external application to an animal, e.g., to treat fleas (non-respirable). The various drugs which are advantageously formulated as described herein are described herein below. The various biologically active agents which may be advantageously formulated to have the physical properties described herein are described in paragraphs herein below.

"Surface viscoelastic modulus" is a measure of the extent that a liquid's surface tension deviates from its original state relative to a perturbation. A low surface viscoelastic modulus represents a fluid surface that resists surface perturbations in an analogous manner to shock absorbers on a car. More precisely, the surface tension changes minimally in response to a change in surface area. In the case of highly conductive formulations, the surface perturbations are thought to result from localized electrical force fluctuations and the rapid creation of new surface as the fluid elongates to form a Taylor cone. A method for determining the viscoelastic modulus is described in detail in WO 2008/094693 the contents of which are incorporated by reference herein.

The surface viscoelastic modulus (E) of the highly conductive aqueous liquid formulations will range from about 0.5 mN/m to about 10 mN/m; preferably from about 2.0 mN/m to about 7.5 mN/m; and more preferably about 5.0 mN/m.

Phase angle is a measure of the time required for the surface to respond to a perturbation. A large phase angle indicates a slower surface response to a perturbation. A slower surface response is considered desirable. In the highly aqueous carrier liquids and formulations described herein, the phase angle will range from about 0.5 degrees to about 90 degrees and preferably from about 10 degrees to about 50 degrees and more preferably about 25 degrees.

Surface tension is a property possessed by liquid surfaces whereby these surfaces behave as if covered by a thin elastic membrane in a state of tension. Surface tension is a measure of the energy needed to increase the surface area of the liquid. Liquids with a lower surface tension will aerosolize more easily than liquids with higher surface tension. Surface tension is measured by the force acting normally across unit length in the surface. The phenomenon of surface tension is due to unbalanced molecular cohesive forces near the surface of a liquid. As this term is used herein, it refers to the surface tension of the liquid formulation in the Taylor Cone just before formation of aerosol droplets.

The surface tension of the aqueous liquid carrier vehicles and formulations is within the range of from about 10 to about 72 milliNewtons/meter. In more preferred embodiments, the surface tension of the aqueous carrier liquids and formulations is within the range of from about 15 to about 45 milliNewtons/meter. In most preferred embodiments, the surface tension is within the range of from about 20 to from about 35 milliNewtons/meter.

Viscosity is the measure of the resistance to fluid flow; thus liquids that flow easily generally have lower viscosity. The viscosity of a liquid composition is not affected significantly by the addition of small amounts of active agent to the composition. However, the addition of certain suspending agents or very high concentration of an active agent can increase the viscosity of the liquid composition. While viscosity may not be a key parameter in forming the aerosols, it will affect the aerosol particle size distribution.

Highly viscous liquids tend to form aerosols having larger particle sizes when aerosolized using an EHD aerosolization means and with more disperse or bimodal distributions. Beyond a critical viscosity value, the formed jet emanating from the Taylor cone will not break up into discreet aerosol particles and instead will form continuous ligaments.

The term "resistivity" refers to the electrical resistance of a material, e.g., the liquid carrier per unit length, area, or volume. While the prior art suggests that a broad resistivity range may be used with EHD spraying devices, i.e., from 10² to 10⁸ Ohm meters, the base carrier liquids being sprayed were non-aqueous or slightly aqueous. The prior art generally suggests that relativity high resistivities of at least 10⁴ Ohm meters should be used to spray highly aqueous liquids.

The inventors have determined that the system 10 shown in **Fig. 1** is especially effective when a highly conductive, highly aqueous liquid composition is supplied to the EHD aerosol apparatus 14. Such highly conductive, highly aqueous liquid compositions comprise three or more basic components: an active agent; liquid carrier vehicle in which the active ingredient may be dissolved or suspended, and a surfactant which provide the formulation with surface rheological properties which enable the production of an aerosol using the EHD aerosol apparatus 14 and optionally, excipients.

Accordingly, embodiments of the invention contemplate that a highly aqueous, highly conductive liquid composition having a surface viscoelastic modulus of from about 0.5 mN/m to about 10 mN/m, a phase angle of from about 0.5 degrees to about 90 degrees, and a conductivity of from about 5.0 µSiemens/cm to about 1000 µSiemens/cm and preferably from about 12.5 µSiemens/cm to about 400 µSiemens/cm will be utilized with the system 10. Another embodiment of the present invention contemplates using a highly aqueous, highly conductive liquid composition having a surface viscoelastic modulus of from about 2.0 mN/m to about 7.5 mN/m, a phase angle of from about 10 degrees to about 50 degrees, and a conductivity of from about 12.5 µSiemens/cm to about 400 µSiemens/cm and preferably from about 12.5 µSiemens/cm to about 400 µSiemens/cm.

Surface tension is a property possessed by liquid surfaces whereby these surfaces behave as if covered by a thin elastic membrane in a state of tension. Surface tension is a measure of the energy needed to increase the surface area of the liquid. Liquids with a lower surface tension will aerosolize more easily than liquids with higher surface tension.

Surface tension is measured by the force acting normally across unit length in the surface. The phenomenon of surface tension is due to unbalanced molecular cohesive forces near the surface of a liquid. As this term is used herein, it refers to the surface tension of the liquid formulation in the Taylor Cone just before formation of aerosol droplets.

In some embodiments of the present invention, the surface tension of the liquid composition is within the range of from about 10 to about 72 milliNewtons/meter. In more preferred embodiments of the present invention, the surface tension of the composition is within the range of from about 15 to about 45 milliNewtons/meter. In most preferred embodiments of the present invention, the surface tension of the composition is within the range of from about 20 to from about 35 milliNewtons/meter.

Viscosity is the measure of the resistance to fluid flow; thus liquids that flow easily generally have lower viscosity. The viscosity of a liquid composition is not affected significantly by the addition of small amounts of active agent to the composition. However, the addition of certain suspending agents or very high concentration of an active agent can increase the viscosity of the liquid composition. Viscosity may not be a key parameter in forming the aerosols, but it does affect particle size distribution. Highly viscous materials tend to form aerosols larger particle sizes and with more disperse or bimodal distributions. Beyond a critical viscosity value, the formed jet emanating from the Taylor cone will not break up into discreet aerosol particles and instead will form continuous ligaments.

The highly conductive, highly aqueous liquid carrier vehicles and formulations described herein will have surface rheological properties described hereinabove. Although, the highly conductive, highly aqueous liquid carrier vehicles and formulations used to produce respirable and non-respirable aerosols are very similar, there are important differences between the aqueous liquids which produce respirable and which produce non-respirable aerosols; accordingly, each will be described separately to avoid confusion.

### Respirable Aerosols From Highly Aqueous Liquid Carrier Vehicles

One embodiment of the invention is directed to a method of delivering a pharmaceutically active agent to the respiratory tract of a patient in need of treatment comprising the steps of:
(a) preparing a liquid carrier vehicle comprising:
   i. from about 50% v/v to about 100% v/v water;
   ii. from about 0% v/v to about 40% v/v ethanol;
   iii. about 0% to about 30% v/v of a co-solvent;
   iv. from about 0.5% to about 10% w/v of a pharmaceutically acceptable excipient; and
   v. from about 0.05% w/v to about 10% w/v of a surfactant;
(b) dissolving or suspending an effective amount of a pharmaceutically active agent in said liquid carrier vehicle to produce a solution or suspension;
(c) producing an aerosol of said solution or suspension using an EHD means having a time varying voltage, wherein the diameter of the aerosol particles is from about 1.0 microns to about 25 microns; and
(d) administering said aerosol to the pulmonary tract of said patient via inhalation of said aerosol;
wherein said liquid formulation formed in Steps (a) and (b) has a surface viscoelastic modulus of from about 0.5 mN/m to about 10 mN/m, a phase angle of from about 0.5 degrees to about 90 degrees, and a conductivity of from about 5.0 µSiemens/cm to about 1000 µSiemens/cm.

A preferred embodiment of the method of delivering a pharmaceutically active agent to the respiratory tract of a patient in need of treatment comprising the steps of:
(a) preparing a liquid carrier vehicle comprising:
   i. from about 70% v/v to about 80% v/v water;
   ii. from about 10% v/v to about 20% v/v ethanol;
   iii. about 10% v/v of a co-solvent;
   iv. from about 0.5% to about 5% w/v of a pharmaceutically acceptable excipient; and
   v. from about 0.3% w/v to about 5% w/v of a surfactant;
(b) dissolving or suspending an effective amount of a pharmaceutically active agent in said liquid carrier vehicle to produce a solution or suspension;
(c) producing an aerosol of said solution or suspension using an EHD means having a time varying voltage, wherein the diameter of the aerosol particles is from about 1.0 microns to about 10 microns; and
(d) administering said aerosol to the pulmonary tract of a patient in need of treatment via inhalation of said aerosol;
wherein said liquid formulation formed in Steps (a) and (b) has a surface viscoelastic modulus of from about 2.0 mN/m to about 7.5 mN/m, a phase angle of from about 10 degrees to about 50 degrees, and a conductivity of from about 10.0 µSiemens/cm to about 400 µSiemens/cm.

For further details regarding the preparation of and components of the highly aqueous carrier liquids and formulations used to produce respirable aerosols, the skilled artisan is directed to the disclosure of US 2003/0185762, the contents are which are incorporated by reference herein.

The term "respiratory tract" as used herein includes the upper airways, including the oropharynx and larynx, followed by the lower airways, which include the trachea followed by bifurcations into the bronchi and bronchioli. The upper and lower airways are called the conductive airways. The terminal bronchioli then divide into respiratory bronchioli, which then lead to the ultimate respiratory zone, the alveoli, or deep lung. Gonda, I. "Aerosols for delivery of therapeutic and diagnostic agents to the respiratory tract," in Critical Reviews in Therapeutic Drug Carrier Systems, 6: 273-313, (1990). Usually, the deep lung, or alveoli, is the primary target of inhaled therapeutic aerosols for systemic delivery. As used herein, the term "respiratory tract" is additionally meant to include administration of the highly aqueous liquid formulations to the nasal passages and to the mucosa of the bucca.

The term "liquid carrier vehicle" as used herein refers to the liquid vehicle in which the drug to be administered is dissolved or suspended. The liquid carrier is "highly aqueous", i.e., it is required to contain at least about 50% water v/v and preferably about 70% v/v water in addition to no more than about 40% v/v ethanol, no more than about 30% v/v of a co-solvent, one or more "pharmaceutically acceptable excipients" and one or more surfactants.

The highly aqueous carrier liquid formulations may include minor amounts, that is, from about 0.5% to about 10% w/v and preferably from about 0.5% to from about 5% w/v of a "pharmaceutically acceptable excipient". Pharmaceutically acceptable excipients are those recognized by the FDA as being safe for use in humans. Additionally, an excipient should have no effect or minimal effect on the sprayability (aerosolizability) of formulations of a drug dissolved or suspended in the aqueous liquid carrier vehicles using the EHD spraying means described above. Additives such as, antioxidants, e.g., Vitamin E, Vitamin E TPGS (α-alpha tocopferol polyethylene glycol 1000 succinate), ascorbic acid, anti-microbials, e.g, parabens, pH adjusting agents, e.g., sodium hydroxide and hydrochloric acid, tonicity adjusting agents, e.g., sodium chloride and viscosity adjusting agents, e.g., polyvinyl pyrrolidone are contemplated for use herein.

From about 0% v/v to about 30% v/v of a co-solvent may be used in the liquid carrier vehicle and preferably from about 2.5% to about 10% v/v will be used and more preferably about 5% v/v. The term "co-solvent" refers to mono- and polyvalent alcohols such as propylene glycol, glycerol, and polyethylene glycol (PEG) having an average molecular weight between about 200 and 4000, preferably between about 200 and 400.

The term "pharmaceutically active agent" refers to biologically active agents that are used for diagnostic purposes as well as agents that are administered to human or animal patients as the active drug substance for treatment of a disease or condition. Such active drug substances are administered to a patient in a "pharmaceutically effective amount" to treat a disease or condition. As would be recognized by one skilled in the art, by "effective amount" is meant an amount of a pharmaceutically active agent having a therapeutically relevant effect on the disease or condition to be treated. A therapeutically relevant effect relieves to some extent one or more symptoms of the disease or condition in a patient or returns to normal either partially or completely one or more physiological or biochemical parameters associated with or causative of the disease or condition. Specific details of the dosage of a particular active drug may be found in its labeling, i.e., the package insert (see 21 CFR §201.56 & 201.57) approved by the United States Food and Drug Administration.

When a pharmaceutically active agent is added to the liquid carrier a solution is produced if the drug is soluble in the liquid carrier and a suspension is produced if the drug is insoluble. The term "suspension" as used herein is given its ordinary meaning and refers to particles of drug or aggregates of particles of drug suspended in the liquid carrier. When the drug is present as a suspension the particles of drug will likely be in the nanometer range.

The surface rheological properties of the highly conductive, highly aqueous carrier liquids and formulations described herein are critical in obtaining stable, mono-modal aerosols of the highly conductive, highly aqueous carrier liquids and formulations when such formulations are aerosolized using the enhanced EHD means described in **Fig. 1** **-** **Fig. 7** herein. Therefore, according to some embodiments of the present invention, a surfactant or multiple surfactants may be added to the active ingredient and carrier liquid to adjust the carrier liquid's surface rheological characteristics.

Surfactants such as natural and synthetic phospholipid derivatives, e.g., lecithin, 1-palmitoyl-2-(16-fluoropallmitoyl)-sn-glycero-3-phosphocholine (DPPC) and 1,2-dimyristoylamido-1,2-deoxyPhophotidylcholine (DDPC); polysorbates, e.g., polyoxyethylene sorbitan monooleate (Tween 80), sorbitan monooleate (Span 80), sorbitan trioleate (Span 85); oleic acid; polyols such as glycerol; medium chain triglycerides; fatty acids; soybean oil; olive oil; sodium dodecyl sulfate (SDS); derivatized carbohydrate surfactants; and combinations of the such surfactants have been found to be useful in the highly conductive, highly aqueous liquid formulations. Derivatized carbohydrate surfactants are preferred for use to prepare carrier liquid formulations which will be aerosolized and inhaled by a patient (respirable aerosol).

The surfactants used should have low animal toxicity and immunogenicity and should be highly effective in lowering surface tension of the highly aqueous liquid carrier vehicle as it is discharged from the EHD spraying means. Further, such surfactant(s) should be used at low concentrations. In general, from about 0.05% to about 10% w/v of a surfactant will be used in the liquid carrier liquid and preferably from about 0.3% to about 5% w/v of the surfactant will be used in the liquid carrier vehicles.

The choice of a particular surfactant for use in a particular liquid carrier vehicle will be made considering the physical and chemical properties of the drug to be aerosolized, e.g. is the drug soluble in water or very insoluble, the amount of ethanol in the liquid carrier vehicle, the nature and amount of any co-solvent or excipient in the liquid carrier vehicle, the desired particle size of the resulting aerosol and the desired spray flow rate.

Derivatized carbohydrate surfactants found to be particularly useful in the highly aqueous liquid carriers are C8-glucose, C9-glucose, The choice of a particular derivatized carbohydrate surfactant for use in a particular liquid carrier vehicle will be made considering the physical and chemical properties of the drug to be aerosolized, e.g. is the drug soluble in water or very insoluble, the amount of ethanol in the liquid carrier vehicle, the nature and amount of any co-solvent or excipient in the liquid carrier vehicle, the desired particle size of the resulting aerosol and the desired spray flow rate.

Derivatized carbohydrate surfactants found to be particularly useful in the highly aqueous liquid carriers are C8-glucose, C9-glucose, C10-glucose, C12-glucose, and C14-maltose and are described further in Table I below. In general, the derivatized carbohydrate surfactants should be selected so that the surfactant is soluble in the carrier liquid. However, the derivatized carbohydrate may be suspended in the carrier liquid and still produce the desired surface tension of from about 20 dyne/cm to about 40 dyne/cm.

The surfactants described herein are capable of effectively reducing the surface tension of the liquid carrier vehicle to a range of from about 20 dyne/cm to from about 40 dyne/cm and preferably from about 25 dyne/cm to about 38 dyne/cm and more preferably from about 25 dyne/cm to about 30 dyne/cm.

**Table I**

| | | **EXEMPLARY CARBOHYDRATE SURFACTANTS** | |
|---|---|---|---|
| **Surfactant** | **Abbreviation For Surfactant** | **Name** | **Molecular Weight** |
| C8-glucose* | S5 | n-octyl-β-D-glucopyranoside | 292.4 |
| C9-glucose** | S2 | n-nonyl-β-D- glucopyranoside | 306.4 |
| C10-glucose* | S3 | decyl-β-D-glucopyranoside | 320.4 |
| C12-glucose** | S4 | n-dodecyl-β-D-glucopyranoside | 348.5 |
| C14-glucose** | S5 | n-tetradecyl-β-D-maltopyranoside | 538.6 |

| | | | |
|---|---|---|---|
| * Available from Sigma-Aldrich (www.sigma-aldrich.com) ** Available from Anatrace (www.anatrace.com) | | | |

The term "aerosol" as used herein refers to a suspension of fine particles (liquid or solid) in air with a range of particle sizes. (See Albert et.al., Comprehensive Respiratory Medicine, 1999, Mosby, London, pp. 7.36.1)

In the case of a respirable aerosol, the particle size of the aerosol droplets produced when the liquid carrier described is sprayed with the EHD device will range from about 1 micron (µm) to about 25 microns in diameter, with the particular size of the aerosol droplet being selected depending on where in the respiratory tract the drug is to be delivered. Generally, if the drug is to be delivered to the deep lung for systemic activity, the particle size of the resulting aerosol will range from about 1 µm to about 5.0 µm and preferably from about 1 µm to about 3.0 µm. If the drug is to be delivered to the mid-lung, the particle size of the resulting aerosol will range from about 3 µm to about 10 µm and preferably from about 5 µm to about 10 µm will be used. If the pharmaceutically active agent is delivered to the oropharangeal region, the buccal about 10 microns (µm) to about 30 microns in diameter.

The respirable highly aqueous liquid compositions described above may be sprayed at relatively fast flow rates, i.e., on the order of 5 to 10 µI/sec. A faster flow rate is important to a patient being treated as faster flow rates translates into less time it takes for the patient to be treated.

Various highly conductive, highly aqueous liquid carrier vehicles were prepared as illustrated by the data shown in Tables II and III. In Tables II -III the following abbreviations are used: E1 is vitamin E TPGS, E2 is polyvinylpyrrolidone, PEG is polyethylene glycol and PG is propylene glycol.

**Table II**

| | Examples of 70% Water/30% Ethanol Carrier Vehicles | | | |
|---|---|---|---|---|
| Surfactant Abbreviation | Amount of Surfactant % (w/v) | Excipient and/or Co-Solvent | Flow Rate (µl/sec) | Resistivity (Ω m) |
| S1 | 0.5 | 10% PEG | 10 | 242 |
| S1 | 0.5 | 0.5% E2 and 10% PG | 8 | 202 |
| S2 | 0.5 | None | 9 | 214 |
| S2 | 0.5 | 0.1% E1 and 10%PG | 8 | 200 |
| S3 | 0.5 | 10% PEG | 5 | 186 |
| S3 | 0.5 | 0.1% E1 and 10% PG | 6 | 214 |
| S4 | 0.1 | 10% PEG | 9 | 493 |
| S4 | 0.1 | 0.1% E1 and 10% PG | 9 | 490 |
| S5 | 0.5 | 0.1% E1 and 10% PG | 8 | 521 |
| S5 | 0.5 | 0.5% E2 and 10%PG | 8 | 206 |

**Table III**

| Examples of 80% Water/20% Ethanol Carrier Vehicles | | | | |
|---|---|---|---|---|
| Surfactant Abbreviation | Amount of Surfactant % (w/v) | Excipient and/or Co-Solvent | Flow Rate (µl/sec) | **Resistivity (Ω · m)** |
| S2 | 0.5 | 0.1% E1 and 10%PG | 7 | **506** |
| S2 | 0.5 | 0. 1% E1 and 10%PG | 10 | **204** |
| S2 | 0.5 | 0.5% E2 and 10%PG | 8 | **201** |
| S2 | 0.5 | 0.5% E2 and 10%PG | 7 | **455** |
| S2 | 0.5 | 0.5% E2 and 10%PG | 7 | **206** |
| S3 | 0.5 | 0.5% E2 and 10%PG | 5 | **200** |
| S3 | 0.1 | 10% PEG | 5 | **205** |
| S3 | 0.1 | 10% PEG | 10 | **205** |
| S3 | 0.5 | 10% PEG | 7 | **219** |
| **S4** | **0.5** | **10% PEG** | **7** | **218** |

The data in Tables II and III illustrates that the highly aqueous, highly conductive liquid carrier vehicles may be aerosolized at relatively fast flow rates, i.e., on the order of 5 to 10 µl per second which translates into a faster treatment time for the patient because the time to inhale the effective dose of the active agent will be shorter.

### Non-Respirable, Highly-Aqueous Liquid Compositions

Another embodiment of the invention is to a method for delivering a biologically-active agent to a target surface in need treatment, which comprises the steps of:
(a) preparing an aqueous liquid carrier vehicle comprising:
   (i) about 60 wt % to about 100 wt % water;
   (ii) about 0 wt % to about 40 wt % of a co-solvent;
   (iii) about 0.05 wt % to about 10 wt % of an acceptable surfactant; and
   (iv) about 0 wt % to about 10 wt % of an excipient;
(b) dissolving or suspending a biologically-effective amount of the biologically-active agent in the liquid carrier vehicle;
(c) producing an aerosol of the solution or suspension using an EHD means having a time varying voltage, wherein the diameter of the aerosol particle is about 60 microns to about 800 microns; and
(d) applying the aerosol to the target surface;
wherein said highly conductive liquid composition has a surface viscoelastic modulus of from about 0.5 mN/m to about 10 mN/m, a phase angle of from about 0.5 degrees to about 90 degrees, and a conductivity of from about 5.0 µSiemens/cm to about 1000 µSiemens/cm.

A preferred embodiment of the method described above is directed to a method for delivering a biologically-active agent to a target surface in need treatment comprising:
(a) preparing an aqueous liquid carrier vehicle comprising:
   (i) about 95 wt % to about 100 wt % water;
   (ii) about 0 wt % to about 5 wt % of a co-solvent;
   (iii) about 0.1 wt % to about 2.5 wt % of an acceptable surfactant; and
   (iv) about 0.1 wt % to about 2.5 wt % of an excipient;
(b) dissolving or suspending a biologically-effective amount of the biologically-active agent in the liquid carrier vehicle;
(c) producing an aerosol of the solution or suspension using an EHD means having a time varying voltage, wherein the diameter of the aerosol particle is about 150 microns to about 350 microns; and
(d) applying the aerosol to the target surface;
wherein said highly conductive liquid composition has a surface viscoelastic modulus of from about 2.0 mN/m to about 7.5 mN/m, a phase angle of from about 10 degrees to about 50 degrees, and a conductivity of from about 10.0 µSiemens/cm to about 400 µSiemens/cm.

In general, the formulations are prepared by adding the components together and mixing to give a liquid solution or solid in liquid suspension. If the active agent is soluble in water, the active agent is mixed with the aqueous liquid and the co-solvent, surfactant, and excipient (if any) are added to the aqueous solution and the mixture is shaken or stirred to produce a homogenous solution. Where the active agent is substantially insoluble in the aqueous liquid component of the carrier vehicle and is soluble in the co-solvent, the active agent is added to the co-solvent, mixed, and the mixture is added to the aqueous component of the aqueous carrier vehicle. Where the active agent is only slightly soluble in water and/or the co-solvent, it may be advantageous to disperse fine particles of the active agent in the liquid carrier vehicle in order to achieve the desired concentration of the active in the carrier vehicle.

The highly-aqueous liquid carrier vehicles and prepared compositions have a resistivity of from about 0.05 ohm-m to about 100 ohm-m, preferably from about 0.1 ohm-m to about 10 ohm-m, and more preferably from about 0.25 ohm-m to about 5 ohm-m. The highly-aqueous liquid carrier vehicles and prepared compositions have a viscosity of from about 0.1 cPs to about 100 cPs, and a surface tension of from about 20 dynes/cm to about 60 dynes/cm.

Unlike the prior art aqueous liquid carrier vehicles, which are generally aerosolized/ sprayed at relatively low flow rates (on the order of µl/sec), the highly-aqueous liquid carrier vehicle maybe sprayed at commercially-acceptable flow rates. As an example, if a multiple site nozzle having ten sites is used to produce an aerosol, and the flow-rate at each site is on the order of 0.5 µl/sec/site to 2.0 µl/sec/site, an overall flow rate of 5 µl/sec to 20 µl/sec would result.

The EHD spraying means described herein above may be used in many configurations in the production of non-respirable aerosols. The device may be stationary or handheld. Such devices are "stationary" in the respect that their size prevents them from being easily held and carried by the user. Stationary EHD devices may be portable if moved on a cart, dolly, or vehicle such as a truck or an airplane. In many of the applications described herein, it is advantageous that the EHD device be small, portable, and handheld. As an example, an EHD device about the size of a cell phone would enable the user/applicator to apply the biologically active aerosols in a variety of locations where it would be inconvenient to move a larger device. For example, a portable, handheld EHD device is ideal for treating one's clothing in a wooded or field environment where there may be deer ticks infected with the bacterium *Borrelia burgdorferi,* which causes Lyme Disease and which is transmitted to humans by the bite of an infected deer tick.

As discussed above, EHD sprayers produce charged particles at the tip of the nozzle. In the case of non-respirable aerosol production, these charged particles can be partially- or fully-neutralized (with, for example, a discharge electrode in the sprayer device). In the case of a non-therapeutic, non-respirable aerosol such as those described in this section, the aerosol is intended to be deposited on a target surface, and an EFET sprayer without means for discharging or with means for only partially discharging an aerosol is preferred since the aerosol would have a residual electric charge as it leaves the sprayer so that the particles would be attracted to and tightly adhere to the target surface.

The term "highly conductive" as used herein, refers to a highly aqueous liquid formulation having the following physical properties: a surface viscoelastic modulus of from about 0.5 mN/m to about 10 mN/m, a phase angle of from about 0.5 degrees to about 90 degrees, and a conductivity of from about 5.0 µSiemens/cm to about 1000 µSiemens/cm and preferably from about 12.5 µSiemens/cm to about 400 µSiemens/cm.

Judicious selection of the surfactant(s) used in the highly aqueous liquid formulations described herein, as well as the selection of the co-solvent will enable one to prepare highly conductive, highly aqueous formulations which can be efficaciously aerosolized using the EHD device described in detail above and as illustrated in **Fig. 1** to **Fig. 7**.

The term "aqueous liquid carrier vehicle" as used herein refers to the liquid carrier vehicle in which the biologically-active agent to be applied to a target surface is dissolved or suspended. The aqueous liquid carrier vehicle is required to contain at least about 60 weight percent to about 100 weight percent water, preferably from about 85 weight percent to about 100 weight percent water, and more preferably from about 90 weight percent to about 100 weight percent water. Aqueous liquid carrier vehicles containing from about 90 weight percent to about 99 weight percent water and more preferably from about 95 weight percent to about 100 weight percent water are "very highly aqueous" liquid carriers

The aerosols can be used to deliver a "biologically-active agent" to a target surface. The term "target surface" as used herein may be any surface that benefits from treatment of a biologically-active agent with a soft cloud of a non-respirable aerosol. As used herein, the term "target surface" does not refer to an interior tissue surface in a human or animal body such as the lungs or oral, vaginal, or rectal cavities. The target surface may be for example, plants, the soil (ground) around plants, the leaves and stems of plants, the eyes, skin, coat, hide, or hide of animals such as cats, dogs, and horses, the skin, eyes, and hair of humans, the clothing of humans, and hard surfaces such as walls, floors, tables, desks, beds, and other furnishings, manufacturing and building infrastructure, and the like found in hospitals, nursing homes, schools, and restaurants. The term "biologically-active agent" refers to an agent or combination of agents that may be used in agriculture, horticulture, veterinary medicine, personal animal, or human care, disinfecting, and other applications where it is desirable to deliver a biologically-active agent to a target surface. The biologically-active agents contemplated for use in the aerosols and methods embodying the invention include but are not limited to herbicides, plant growth regulators, insecticides, fungicides, miticides, biocides, antibacterials, antivirals, anti-inflammatories, disinfectants, ocular decongestants, skin and eye treatments, and the like.

Illustrative, but non-limiting examples of the aerosols prepared as described herein are aerosols useful to deliver insecticides and fungicides to trees and shrubs, plants such as roses, orchids, violets, and other valuable flowering plants, as well as to deliver herbicides to bed plantings and home gardens, especially when handheld, battery powered, portable EHD devices are used to produce the aerosol. The aerosols and methods embodying the invention can be used to apply anti-tick, flea, and mite active agents to the coat of mammals such as dogs, cats, and horses, the skin and hair of humans, and the outer clothing of humans to protect against fleas, ticks, and mites. The aerosols can be used to apply disinfectant agents to hard surfaces in schools, restaurants, hospitals, businesses, stores, manufacturing facilities, and the home. In schools, for example, the aerosols may be use to treat desks and cafeteria tables to prevent the spread of viruses and bacterial, especially in influenza season.

Illustrative, but non-limiting examples of specific biologically-active agents useful in the aerosols and methods embodying the invention include: herbicides e.g., (2,4,5-trichlorophenoxy)acetic acid, (4-chloro-2-methylphenoxy)acetic acid, (2,4-dichlorophenoxy)acetic acid, 4-(4-chloro-o-tolyloxy)butyric acid, fluazifop-p-butyl (Ornamec^{®}, Gordon Corp, Kansas City, Mo.), pelargonic acid (Scythe^{®}, Mycogen Corp., San Diego, Calif.), and isopropylamine salt of N-(phosphonomethyl)glycine (Roundup^{®}, Scotts, Marysville, Ohio or Glyphomax^{®}, Dow Agrosciences, Indianapolis, Ind.); fungicides e.g., manganese ethylene bisdithiocarbamate (Maneb), 1-(4-chlorophenoxy)-3,3-dimethyl-1-(1H-1,2,4-triazol-1-yl)-2-butanone (Strike^{®}, Olympic Horticultural Products, Mainland, Pa.), azoxystrobin (Amistar^{®}, Syngenta, Basel, CH), andtrifloxystrobin (Compass™, Bayer CropScience, Research Triangle Park, N.C.); insecticides, e.g., *Bacillus thuringiensis* (B.t.) (sold under the trade names Dipel^{®} (Valent Corp, Dublin, Calif.), Thuricide^{®} (Bonide Products, Oriskany, N.Y.), Bactospeine^{®} (PBI/Gordon, Kansas City, Mo.), Leptox, Novabac, and Bug Time); synthetic pyrethroids, e.g., permethrin, cypermethrin, fenvalerate/esfenvalerate, tralomethrin, bifenthrin, cyfluthrin, and lambda-cyhalothrin, O,O-Diethyl 0-(2-isopropyl-6-methyl-4-pyrimidinyl) phosphorothioate (diazinon); treatments for fleas, ticks, and lice, e.g., lindane and malathion (headlice, pubic lice), permethrin (ticks), N,N-diethyl-meta-toluamide (DEET) (mosquitoes), fenthion, and cythioate (fleas); and disinfectants, e.g., 3,4',5 tribromosalicylanilide (tribromsalan).

The biologically-active agents described herein are present in the aqueous liquid carrier vehicles at a "biologically-effective amount". As would be recognized by one skilled in the art, by "biologically-effective amount" is meant an amount of a biologically-active agent that is sufficient to provide the result sought. In general, from about 0.01 weight percent to about 50 weight percent of the biologically-active agent will be present in the liquid carrier vehicle. Specific details of the effective dosage or concentration of a particular active agent may be found in its product labeling, e.g., the package insert if the active agent is regulated by the United States Food and Drug Administration (FDA) (see, 21 CFR § 201.56 & 201.57) or the labeling approved by the United States Environmental Protection Agency (EPA) if the active agent is, e.g., a herbicide, insecticide, miticide, and the like, which is covered by the rules and regulations of the EPA.

When a biologically-active agent is added to the aqueous liquid carrier vehicle, a solution is produced if the active agent is soluble in the liquid carrier vehicle and a suspension is produced if the active agent is insoluble. The term "suspension" as used herein is given its ordinary meaning and refers to particles of active agent or aggregates of particles of active agent suspended in the liquid carrier vehicle. When the active agent is present as a suspension the particles of active agent will preferably be in the nano or micron size range.

Among the advantages of embodiments of the present invention is the ability to use a highly-aqueous carrier liquid that is more "bio-friendly" than conventional carriers used in EHD spraying such as oil-based or solvent-based carriers.

Depending on the biologically-active agent used in the aerosols and methods embodying the invention, it may be advantageous to include a co-solvent in the aqueous liquid carrier vehicle. The co-solvent may be selected from such groups as alcohols, ethers, alkyl sulfoxides, and propylene oxides. Examples of specific co-solvents include ethanol, 2-ethylhexanol, diacetone alcohol, diisobutyl ketone, isobutanol, isophorone, methyl isobutyl ketone, n-butanol, n-pentanol, n-propanol, and combinations thereof. Ethanol is a particularly preferred solvent because it is soluble in water, is relatively inexpensive, and is safe for the environment, animals, and humans.

The choice of a particular co-solvent or mixture of co-solvents is within the skill of the art and will be made by the skilled artisan taking into account such factors as how an aerosol will be used, the particular active agent, and if the target surface is a plant, animal, or hard surface. The co-solvent should be soluble in or miscible with water, have a viscosity in the range of 0.1 cPs to 100 cPs, and should not raise the surface tension of the liquid carrier vehicle above 60 dynes/cm. The co-solvent will be present in the liquid carrier vehicle described in this section at from about zero weight percent to about 40 weight percent, preferably from about 1 weight percent to about 40 weight percent, and more preferably from about 5 weight percent to about 15 weight percent.

An essential component of the highly-aqueous liquid carrier vehicle is the surfactant. It important that the surfactant selected be capable of quickly lowering surface tension at the interface between air and liquid as the liquid is exiting the EHD spray nozzle and the electric charge is being applied to the liquid to form the aerosol droplets. While not being bound by theory, the choice of surfactant or mixtures of surfactants used in the liquid carrier vehicles described herein, it is important to control the surface tension as the aerosol droplet is formed coming from the EHD spray nozzle. It is desirable to keep the surface tension as low as possible at this point in order to produced good aerosolization of the aqueous liquid.

Carrier vehicles described in this section should be non-corrosive to the EHD device, should be environmentally safe at the concentrations used, should be non-toxic to humans and animals at the concentrations used, and should have no adverse effect on the activity of the biologically-active agent being delivered in the aerosols.

Examples of surfactants found to be useful in the aerosols and liquid carrier vehicles are nonionics such as alkoxypoly(ethyleneoxy) alcohols such as Rhodasurf^{®} BC 720 (Brenntag, Antwerp, BE), a water-soluble alkoxypoly(ethyleneoxy) ethanol surfactant having an HLB (Hydrophile-Liphophile Balance) of 13.8, alkyl polyglycosides sold under the tradenames Agnique^{®} PG 8107-U (HLB 13.6) and Agnique^{®} PG 9116 (HLB 13.1) (both from Cognis Corp., Cincinnati, Ohio); polyoxyethylene ethers, e.g., polyoxyethylene(10) tridecyl ether (ANAPOE^{®}-C₁₂E₁₀, Anatrace, Maumee, Ohio); alkyl-β-D-glucopyranosides, e.g., hexyl-, heptyl-, octyl-, decyl-, and dodecyl-β-D-glucopyranoside; and alkyl-(3-D-maltoglucopyranosides, e.g., hexyl-, octyl-, nonyl-, decyl-, undecyl- , dodecyl-, and tetradecyl-(3-D-maltoglucopyranoside (Anatrace).

The choice of a particular surfactant for use in a particular liquid carrier vehicle will be made considering the physical and chemical properties of the active agent to be aerosolized, e.g. whether the active agent is soluble in water or very insoluble, the amount of co-solvent in the liquid carrier vehicle, the nature and amount of any excipient in the liquid carrier vehicle, the desired particle size of the resulting aerosol and the desired spray flow rate. The surfactant will be present in the liquid carrier vehicle at from about 0.05 weight percent to about 10 weight percent, preferably from about 0.05 weight percent to about 5 weight percent, and more preferably from about 0.1 weight percent to about 2.5 weight percent.

Other optionally-present components in the aerosols and aqueous liquid carrier vehicles are "biologically-acceptable excipients". A used herein, the term "biologically-acceptable excipients" include those compounds and additives listed by the FDA as being generally recognized as safe (GRAS) for use in humans (see, 21 CFR § 182). The term also includes those additives that are exempted from the requirement of a tolerance when used in accordance with good agricultural practices. See Federal Insecticide, Fungicide and Rodenticide Act (FIFRA), 7 U.S.C. §136 et seq. (1996) and 40 C.F.R. § 180.1001.

Illustrative of such excipients include but not limited to polyols e.g., propylene glycol, glycerol, polyvinyl alcohol (PVA), and polyethylene glycol (PEG) having an average molecular weight between about 200 and 4000, antioxidants, e.g., Vitamin E, Vitamin E TPGS (alpha-tocopferol polyethylene glycol 1000 succinate), ascorbic acid, anti-microbials, e.g., parabens, pH-adjusting agents, e.g., sodium hydroxide and hydrochloric acid, viscosity-adjusting agents, e.g., polyvinylpyrrolidone, and ionic materials to add charge to the liquid carrier formulation are contemplated for use herein.

While the selection of any particular biologically-acceptable excipient or mixture of excipients is within the skill of the art, the decision regarding whether to add an excipient, and if so which one, will be made taking into account the purpose of the excipient in a specific aqueous liquid carrier vehicle. Any excipient used in the aerosols or liquid carrier vehicles described herein should have no effect or minimal effect on the sprayability of the aqueous liquid containing the biologically-active agent.

The particle size of the aerosol droplets should be sufficiently large to ensure that the aerosol particles will not be inhaled by an animal or human. The particle size of the aerosol droplets should average from about 60 microns in diameter to about 800 microns in diameter, preferably from about 80 µm to about 500 µm, and more preferably about 150 µm to about 350 µm in diameter. The average particle size of the droplets is usually referred to as "mass median diameter" (MMD). It is also important that the corresponding geometric standard deviation (GSD) be low, indicating a monodisperse or nearly monodisperse aerosol. A polydisperse aerosol will contain many aerosol particles that are smaller than the target range and many that are larger. Aerosol particles smaller than about 50 µm in diameter might be inhaled or "respired" by animals or humans as the aerosol is being applied to the target surface. On the other hand, if the aerosol particles are larger than about 800 µm the aerosol droplets can coalesce and drip off the target surface wasting the biologically-active agent. It is thus highly desirable that the aerosol be as nearly monodisperse as possible.

In general, the formulations are prepared by adding the components together and mixing to give a liquid solution or solid in liquid suspension. If the active agent is soluble in water, the active agent is mixed with the aqueous liquid and the co-solvent, surfactant and excipient (if any) are added to the aqueous solution and the mixture is shaken or stirred to produce a homogenous solution. Where the active agent is substantially insoluble in the aqueous liquid component of the carrier vehicle and is soluble in the co-solvent, the active agent is added to the co-solvent, mixed and the mixture is added to the aqueous component of the aqueous carrier vehicle. Where the active agent is only slightly soluble in water and/or the co-solvent it may be advantageous to disperse fine particles of the active agent in the liquid carrier vehicle in order to achieve the desired concentration of the active in the carrier vehicle.

In the examples shown below, various abbreviations and trade names are used. The following Table IV provides a description of the trade names and abbreviations used.

**Table IV**

| Term | Product | Manufacturer |
|---|---|---|
| WBG | WEED-B-GON® Ready-to-use weed killer containing 0.20% 2,4-dichlorophenoxyacetic acid, dimethylamine salt and 2-(2-methyl-4-chlorophenoxy) propionic acid, dimethylamine salt as the active agents | The Ortho Group P.O. Box 1749, Columbus, OH 43216 U.S.A. |
| Emulphogen Now known as Rhodasurf BC 720 | Rhodasurf® BC 720 is a water-soluble alkoxypoly(ethyleneoxy)ethanol surfactant having an HLB of 13.8 | Rhodia, 26, quai Alphonse Le Gallo 92512, boulogne-Billancourt Cedex, France. |
| PBS | Phosphate Buffer Solution | |
| C-10 | octyl-β-D- glucopyranoside | Anatrace, 434 West Dussel Drive, Maumee, OH 43537 U.S.A. |
| 2,4-D DMA | 2,4-dichlorophenoxyacetic acid, dimethylamine salt | |
| A-PG9116 | Agnique PG 8107-U nonionic alkyl polyglycoside Having an HLB of 13.1 | Cognis Corp. USA, 5051 Estecreek Drive, Cincinnati, OH 45232-1446 U.S.A. |
| A-PG8107-U | Agnique PG 8107-U nonionic alkyl polyglycoside Having an HLB of 13.6 | Cognis Corp. USA, 5051 Estecreek Drive, Cincinnati, OH 45232-1446 U.S.A. |

**Table V**

| Examples of Highly Aqueous formulations | | | |
|---|---|---|---|
| **Composition (wt %)** | **Surface Tension (dyne/cm)** | **Viscosity (cP)** | **Flow Rate (µl/s/site)** |
| 10.9%WBG 22.35% EtOH 65.76% H₂O 1% C-10 | 36.9 | ND | 1.04 |
| 99% WBG 1% C-10 | 39.3 | 1.96 | 1.25 |
| 99% WBG 1% A-PG8107-U | 37.2 | 1.86 | 1.04 |
| 99% WBG 1% A-PG9116 | 33.8 | 1.86 | 1.04 |
| 99% PBS 1% C-10 | 29.1 | 1.00 | 0.83 |
| 3.05% DMA salts of 2,4-D 95.9% PBS 1% C-10 | 37.5 | 1.71 | 1.04 |
| 3.05% DMA salts of 2,4-D 95.9% H₂O 1% C-10 | 36.8 | 1.66 | 1.04 |
| 99% WBG 1% Emulphogene | 38.0 | 1.68 | 1.04 |
| 99% WBG 1% Desonic DA-4 | 35.5 | 1.73 | 1.25 |
| 99% WBG 0.9% A-PG8107-U 0.1% C-10 | 36.6 | 1.73 | 0.83 |

Further details regarding the method and aqueous liquid carrier vehicles used in methods embodying this invention, as well as numerous examples are described in US 2008/0259519 A1 the contents are incorporated by reference herein.

In some embodiments of this invention, the surface rheological properties of the liquid composition comprise: a surface viscoelastic modulus that is preferably less than 10 mN/m, and a Phase angle that is greater than 10 degrees while the conductivity is between 12.5 and 1000 µSiemens/cm and preferably from about between 12.5 µSiemens/cm to about 400 µSiemens/cm. In other embodiments, it may be possible to achieve a liquid composition with physical properties falling within these parameters by simply combining the active ingredient and an appropriate liquid carrier vehicle. However, if the combination of the active ingredient and the liquid carrier vehicle does not produce a highly aqueous liquid formulation (composition) having physical properties falling within these parameters, the addition of surfactant(s) to the liquid carrier vehicle will bring the composition within the required parameters.

Depending on the application in which the non-respirable aerosols are used, additional formulation excipients may be included in the liquid formulation. Such materials may be included for a variety of purposes including but not limited to: stabilization of the highly aqueous, highly conductive liquid formulation; facilitating control of aerosol particle size; increasing the solubility of the active ingredient in the liquid carrier vehicle; lowering the surface tension of the liquid carrier vehicle; and antimicrobial and antioxidant materials. As would be recognized by those skilled in the art, additional ingredients may be added as long as the resulting highly aqueous, highly conductive liquid formulation has the following critical properties: i.e., a surface viscoelastic modulus of from about 0.5 mN/m to about 10 mN/m, a phase angle of from about 0.5 degrees to about 90 degrees, and a conductivity of from about 5.0 µSiemens/cm to about 1000 µSiemens/cm and preferably from about 12.5 µSiemens/cm to about 400 µSiemens/cm.

Once solubilized or suspended, the active ingredient should also be stable in the liquid carrier itself, and stable in the final formulation. Stability requires that the active ingredient not lose activity prior to aerosolization (i.e., retains a reasonable shelf-life), and that the active ingredient not lose activity or degrade significantly as a result of the process of aerosolization. Furthermore, in some applications it is required that the highly aqueous, highly conductive liquid composition be stable over time. In various embodiments, stability issues can be addressed by the addition of a stabilizing ingredient to the composition.

One or more of the following ingredients may be added to the formulations to increase physical stability of the composition: oils, glycerides, polysorbates, celluloses lecithin, polyvinyl pyrrolidone, polyethyl glycol, saccharide gums, and alginates. In some embodiments, antioxidants such as ascorbic acid and ascorbic acid esters, Vitamin E, tocopherols, butylated hydroxyanisole, and butylated hydroxytoluene may be added to reduce degradation of an active agent such as a drug caused by oxidation. In some embodiments of the present invention, chelating or complexing agents such as citric acid, cyclodextrins, and ethylenediaminetetracetic acid may be added (as an alternative or in addition to the stabilizing agents just described) to stabilize drug compositions and to increase the solubility of the active ingredient in the composition.

Alternatively or additionally, in some embodiments preservative ingredients may be added to the composition to maintain the microbial integrity of the highly conductive composition. For example, in some embodiments of the present invention, at least one of the following ingredients is added to preserve compositions against microbial contamination or attack: benzalkonium chlorides, phenol, parabens, or any other acceptable antimicrobial or antifungal agent.

Both respirable and non-respirable aerosols may be produced using methods embodying the invention. The key feature of both types of aerosols is the control of surface rheological properties of the highly aqueous liquid carrier vehicle. The combination of modifying surface rheology and superimposing a sinusoidal waveform onto the direct current (DC) electrical field enables electrohydrodynamic aerosolization of high aqueous content formulations (>50% water) beyond what can be achieved through other means, including the individual gains by applying surface rheology modification and superimposing a sinusoidal waveform onto the DC electrical field individually. It was unanticipated that the combination of surface rheology modification and superimposing a sinusoidal waveform onto the DC electrical field would produce a benefit greater than the sum of the individual benefits.

Surface rheology modifications, via well reasoned and portioned combinations of surfactants and excipients successfully, reduce the dynamic surface tension and the overall surface visco-elastic modulus while also increasing the viscous property of the Taylor cone's liquid-air interface. The above changes within the Taylor cone are believed to harmonize the fluid dynamics and electrical dynamics at the liquid - air interface, a requirement for successful electrohydrodynamic aerosolization cone jet mode operation.

Superimposing a time varying waveform, which is preferably sinusoidal, onto the direct current (DC) electrical field disrupts hydrogen bonding in water. Hydrogen bonding is well known to impart the uniquely high surface tension and permittivity. Thus, the superimposition of the time varying waveform onto the DC electrical field reduces the surface tension of the liquid being sprayed. Reducing surface tension and permittivity harmonizes the fluid dynamics and electrical dynamics at the liquid - air interface, enabling cone-jet operation.

The successful use of embodiments of the present is demonstrated by the experimental results data shown in Table VI below which presents raw data obtained when solutions having different percent water content are subjected to aerosolization with the system 10 shown in **Fig. 1**. Of interest is row 8, which illustrates the results obtained for a 70 percent water solution without superposition of the time-varying voltage upon the DC voltage supplied to the EHD apparatus 14, and rows 9 through 13, where the time-varying voltage was superimposed upon the DC voltage. As shown in the fifth column of the data, the DC voltage was varied over a range of 8.9 to 11.1 Kv for the tests. Data in Table VII, labeled "Inventive Data Averaged" compares the data in row 8, without superposition of the time-varying voltage, to the average of the data in rows 9 through 13, with superposition of the time-varying voltage. Of prime importance is the fine particle dose which is seen to have increased from 21.6 percent to 25.9 percent, which is a fine particle dose improvement of 19.8 percent.

Table VIII, labeled "Inventive Best Data" compares the data in row 8, without superposition of the time-varying voltage, to the best data in row 10, with superposition of the time-varying voltage onto 9.0 Kv DC voltage. Again the fine particle dose increases, from 21.6 percent to 26.6 percent for fine particle dose improvement of 23.1 percent. The improvements shown in the fine particle dose are therapeutically and economically meaningful.

**Table VI**

| Raw Data | | | | | | | | |
|---|---|---|---|---|---|---|---|---|
| Row No. | % H₂O (Vol %) | % EtOH (190 Proof) (Vol %) | Surfactants To Adjust Surface Rheology | AC Superposition | Voltage (kV) | Emitted Dose (%) | Fine Particle Fraction (%) | Fine Particle Dose (%) |
| 1 | 0 | 100 | No | No | 10.8 | 88.5 | 91.9 | 81.3 |
| 2 | 20 | 80 | No | No | 10.8 | 70.5 | 85.4 | 60.2 |
| 3 | 25 | 75 | No | No | 10.8 | 70.2 | 83.6 | 58.7 |
| 4 | 30 | 70 | No | No | 10.8 | 62.8 | 84.7 | 53.2 |
| 5 | 40 | 60 | No | No | 10.6 | 58.7 | 84.4 | 49.5 |
| 6 | 50 | 50 | No | No | 9.8 | 42.0 | 77.2 | 32.4 |
| 7 | 60 | 40 | No | No | 9.0 | 39.9 | 71.0 | 28.3 |
| 8 | 70 | 30 | No | No | 8.7 | 29.4 | 73.5 | 21.6 |
| 9 | 70 | 30 | No | Yes | 10.3 | 73.0 | 25.0 | 18.3 |
| 10 | 70 | 30 | Yes | Yes | 11.1 | 79.0 | 29.6 | 23.4 |
| 11 | 70 | 30 | Yes | Yes | 9.0 | 54.5 | 48.8 | 26.6 |
| 12 | 70 | 30 | Yes | Yes | 9.3 | 48.5 | 50.7 | 24.6 |
| 13 | 70 | 30 | Yes | Yes | 8.9 | 60.2 | 40.5 | 24.4 |
| 14 | 70 | 30 | Yes | Yes | 10.4 | 87.4 | 26.7 | 23.3 |
| 15 | 80 | 20 | No | No | 7.9 | 10.9 | 82.9 | 9.0 |
| 16 | 90 | 10 | No | No | 7.6- 9.0 | N/A | N/A | N/A |
| 17 | 100 | 0 | No | No | 7.6 ― 9.0 | N/A | N/A | N/A |

**Table VII**

| Inventive Data Averaged | | | | | | | |
|---|---|---|---|---|---|---|---|
| Row No. | % H₂O (Vol %) | % EtOH (190 Proof) (Vol %) | Surfactants To Adjust Surface Rheology | AC Superposition | Emitted Dose (%) | Fine Particle Fraction (%) | Fine Particle Dose (%) |
| 1 | 70 | 30 | No | No | 29.4 | 73.5 | 21.6 |
| 2 | 70 | 30 | Yes | Yes | 65.9 | 39.3 | 25.9 |
| | | | | | | | Fine Particle Dose Improvement (%) = 19.8% |

**Table VIII**

| Inventive Element Best Data | | | | | | | |
|---|---|---|---|---|---|---|---|
| Row No. | % H₂O (Vol %) | % EtOH (190 Proof) (Vol %) | Surfactants To Adjust Surface Rheology | AC Superposition | Emitted Dose (%) | Fine Particle Fraction (%) | Fine Particle Dose (%) |
| 1 | 70 | 30 | No | No | 29.4 | 73.5 | 21.6 |
| 2 | 70 | 30 | Yes | Yes | 54.5 | 48.8 | 26.6 |
| | | | | | | | Fine Particle Dose Improvement (%) = 23.1% |

While embodiments of invention have been illustrated and described as utilizing a highly conductive liquid composition, the invention also contemplates applications that utilize other liquid compositions, since anything that is soluble is also conductive. Thus the system 10 also may utilize the highly aqueous liquid carrier formulations described in published US Patent Application No. 2003/0185762, which is incorporated herein by reference.

An embodiment of the invention is directed to an improved electrohydrodynamic (EHD) apparatus having a time varying voltage component. Embodiments of the invention further relates to the use of such enhanced EHD apparatus to produce respirable and non-respirable aerosols from highly aqueous liquids, as well as the use of such aerosols. The combination of modifying surface rheology and superimposing a time varying waveform onto the direct current (DC) electrical field enables electrohydrodynamic aerosolization of high aqueous content formulations (>50% water) beyond what can be achieved through other means, including the individual gains by applying surface rheology modification and superimposing a sinusoidal waveform onto the DC electrical field individually.

In accordance with the provisions of the patent statutes, the principle and mode of operation of embodiments of the invention have been explained and illustrated in its preferred embodiment. However, it must be understood that this invention may be practiced otherwise than as specifically explained and illustrated without departing from its spirit or scope.

## Claims

1. An aerosolization system comprising:
an aerosol generating device for aerosolization of a liquid; and
a voltage supply connected to said aerosol generating device, said voltage supply operative to generate a voltage including a high voltage DC component and a time-varying component.

2. The system according to claim 1 also including a high voltage DC supply and an AC voltage supply, the DC and AC voltage supplies being connected through a coupling circuit to said connected aerosol generating device;
optionally further including a buffer circuit connected between said AC voltage supply and said coupling circuit;
optionally said AC voltage supply provides a voltage having a magnitude within a range of two to five volts and a frequency within a range of 75 kHz to 110 kHz and further wherein the DC voltage supply provides a voltage having a magnitude within a range of of 3 Kv to 30 Kv.

3. The system according to any preceding claim wherein said aerosol generating device is an electrohydrodynamic aerosolization device.

4. The system according to claim 3 further including:
a) a supply of a liquid for aerosolization within said aerosol generating device with said liquid being a highly aqueous, highly conductive liquid composition having a surface viscoelastic modulus of from about 0.5 mN/m to about 10 mN/m, a phase angle of from about 0.5 degrees to about 90 degrees, and a conductivity of from about 5.0 µSiemens/cm to about 1000 µSiemens/cm, optionally said surface viscoelastic modulus of said highly aqueous, highly conductive liquid composition ranges from about 2.0 mN/m to from about 7.5 mN/m; or
b) a supply of a liquid for aerosolization within said aerosol generating device with said liquid being a liquid carrier vehicle for a dissolved or suspended active agent; wherein said liquid carrier vehicle has a resistivity of from about 25 ohm m to about 8000 ohm m and a surface tension of from about 20 dyne/cm to from about 40 dyne/cm.

5. A method for generating an aerosol comprising the steps of:
(a) providing an aerosol generating device;
(b) applying a voltage that includes a high voltage DC component and a time varying component to the aerosol generating device; and
(c) supplying a liquid to the aerosol generating device with the generating device and the voltage co-operating to generate an aerosol from the liquid.

6. The method according to claim 5 wherein the aerosol generating device provided in step (a) is an electrohydrodynamic aerosolization device; optionally the liquid supplied in step (c) is a highly aqueous, highly conductive liquid composition having a surface viscoelastic modulus of from about 0.5 mN/m to about 10 mN/m, a phase angle of from about 0.5 degrees to about 90 degrees, and a conductivity of from about 5.0 µSiemens/cm to about 1000 µSiemens/cm, optionally the liquid composition has a surface viscoelastic modulus of from about 2.0 mN/m to about 7.5 mN/m, a phase angle of from about 10 degrees to about 50 degrees, and a conductivity of from about 10 µSiemens/cm to about 400 µSiemens/cm, optionally the liquid composition has a surface viscoelastic modulus of about 5.0 mN/m, a phase angle of about 25 degrees, and a conductivity of from about 50 µSiemens/cm to about 90 µSiemens/cm.

7. A method of delivering a pharmaceutically active agent to the respiratory tract of a patient in need of treatment comprising the steps of:
(a) preparing a liquid carrier vehicle comprising:
i. from about 50% v/v to about 100% v/v water;
ii. from about 0% v/v to about 40% v/v ethanol;
iii. about 0% to about 30% v/v of a co-solvent;
iv. from about 0.5% to about 10% w/v of a pharmaceutically acceptable excipient; and
v. from about 0.05% w/v to about 10% w/v of a surfactant;
(b) dissolving or suspending an effective amount of a pharmaceutically active agent in said liquid carrier vehicle to produce a solution or suspension;
(c) producing an aerosol of said solution or suspension using an EHD means having a time varying voltage, wherein the diameter of the aerosol particles is from about 1.0 microns to about 25 microns; and
(d) administering said aerosol to the pulmonary tract of said patient via inhalation of said aerosol;
wherein said liquid formulation formed in Steps (a) and (b) has a surface viscoelastic modulus of from about 0.5 mN/m to about 10 mN/m, a phase angle of from about 0.5 degrees to about 90 degrees, and a conductivity of from about 5.0 µSiemens/cm to about 1000 µSiemens/cm.

8. The method according to claim 7 wherein said liquid carrier vehicle component of said liquid formulation comprises:
i. from about 70% v/v to about 80% v/v water;
ii. from about 0% v/v to about 30% v/v ethanol;
iii. about 0% to about 30% v/v of a co-solvent;
iv. from about 0.5% to about 5.0% w/v of a pharmaceutically acceptable excipient; and
v. from about 0.3% w/v to about 5.0% w/v of a surfactant.

9. The method according to claim 7 or 8, wherein at least one of:
said liquid formulation has a surface viscoelastic modulus of from about 2.0 mN/m to about 7.5 mN/m, a phase angle of from about 10 degrees to about 50 degrees, and a conductivity of from about 10 µSiemens/cm to about 400 µSiemens/cm; and/or
said surfactant is a derivatized carbohydrate; and/or
said surfactant is a derivatized carbohydrate selected from the group consisting of n-octyl-β-D-glucopyranoside, n-nonyl-β-D-glucopyranoside, decyl-β-D-glucopyranoside, n-dodecyl-β-D-glucopyranoside, and n-tetradecyl-β-D-maltopyranoside; and/or
said co-solvent is selected from the group consisting of propylene glycol, glycerol and polyethylene glycol, optionally said co-solvent is group consisting of propylene glycol; and/or
said pharmaceutically acceptable excipient is selected from the group consisting of antioxidants, antimicrobials, pH adjusting acids and bases, tonicity adjusting agents and viscosity adjusting agents; and/or
said aerosol particle size is from about 1.0 microns to about 3.0 microns.

10. The method according to claim 7 wherein
the liquid carrier vehicle comprises:
i. from about 70% v/v to about 80% v/v water;
ii. from about 10% v/v to about 20% v/v ethanol;
iii. about 10% v/v of a co-solvent;
iv. from about 0.5% to about 5% w/v of a pharmaceutically acceptable excipient; and
v. from about 0.3% w/v to about 5% w/v of a surfactant;
the diameter of the aerosol particles is from about 1.0 microns to about 10 microns; and said liquid formulation has a surface viscoelastic modulus of from about 2.0 mN/m to about 7.5 mN/m, a phase angle of from about 10 degrees to about 50 degrees, and a conductivity of from about 10.0 µSiemens/cm to about 400 µSiemens/cm.

11. The method according to claim 10, wherein said con-solvent is selected from the group consisting of propylene glycol, glycerol and polyethylene glycol and wherein said surfactant is a derivatized carbohydrate surfactant selected form the group consisting of n-octyl-β-D-glucopyranoside, n-nonyl-β-D-glucopyranoside, decyl-β-D-glucopyranoside, n-dodecyl-β-D-glucopyranoside, and n-tetradecyl-β-D-maltopyranoside.

12. A method for delivering a biologically-active agent to a target surface in need treatment, which comprises the steps of:
(a) preparing an aqueous liquid carrier vehicle comprising:
(i) about 60 wt % to about 100 wt % water;
(ii) about 0 wt % to about 40 wt % of a co-solvent;
(iii) about 0.05 wt % to about 10 wt % of an acceptable surfactant; and
(iv) about 0 wt % to about 10 wt % of an excipient;
(b) dissolving or suspending a biologically-effective amount of the biologically-active agent in the liquid carrier vehicle;
(c) producing an aerosol of the solution or suspension using an EHD means having a time varying voltage, wherein the diameter of the aerosol particle is about 60 microns to about 800 microns; and
(d) applying the aerosol to the target surface;
wherein said highly conductive liquid composition has a surface viscoelastic modulus of from about 0.5 mN/m to about 10 mN/m, a phase angle of from about 0.5 degrees to about 90 degrees, and a conductivity of from about 5.0 µSiemens/cm to about 1000 µSiemens/cm.

13. The method according to claim 12, wherein at least one of:
said highly conductive liquid composition has a surface viscoelastic modulus of from about 2.0 mN/m to about 7.5 mN/m, a phase angle of from about 10 degrees to about 50 degrees, and a conductivity of from about 10.0 µSiemens/cm to about 400 µSiemens/cm; and/or
the diameter of the aerosol particle is about 100 microns to about 350 microns; and/or
the concentration of the biologically-active agent in the liquid carrier vehicle is about 0.1 wt% to about 30 wt%; and/or
said biologically- active agent is selected from the group consisting of herbicides, plant growth regulators, insecticides, fungicides, miticides, biocides, antibacterials, anti-virals, topical antihistamines, and disinfecting agents; and/or
said liquid carrier vehicle contains from about 70% to about 80% water; and/or
said liquid carrier vehicle contains from about 1 wt % to about 30 wt% of a co-solvent, optionally said liquid carrier vehicle contains from about 5 wt% to about 15 wt% of a co-solvent, optionally said co-solvent is selected form the group consisting of ethanol, 2-ethylhexanol, diacetone alcohol, diisobutyl ketone, isobutanol, isophorone, methyl Isobutyl ketone, n-butanol, n-pentanol, n-propanol, and combinations thereof, optionally said co-solvent is ethanol; and/or
said liquid carrier vehicle contains from about 0.05 wt% to about 5 wt% of a surfactant, optionally said liquid carrier vehicle contains from about 0.1 wt% to about 2.5 wt% of a surfactant, optionally said liquid carrier vehicle contains about 1 wt% of a surfactant, and/or said surfactant is selected from the group consisting of an alkyl polyglycoside, a polyoxyethylene ether, an alkyl-β-D-glucopyranoside, and an alkyl-β-D-maltoglucopyranoside.

14. The method according to Claim 12 or 13, wherein said liquid carrier vehicle has a resistivity of from about 2.5 Ωm to about 5 Ωm; wherein said liquid carrier vehicle has a viscosity of from about 1.5 cPs to about 40 cPs; and wherein said liquid carrier vehicle has a surface tension of from abut 20 dyne/cm to about 40 dyne/cm; optionally said liquid carrier vehicle has a resistivity of from about 2.5 Ωm to about 5 Ωm; wherein said liquid carrier vehicle has a viscosity of from about 1.5 cPs to about 40 cPs; and wherein said liquid carrier vehicle has a surface tension of from abut 20 dyne/cm to about 40 dyne/cm; optionally said highly conductive liquid composition has a surface viscoelastic modulus of from about 2.0 mN/m to about 7.5 mN/m, a phase angle of from about 10 degrees to about 50 degrees, and a conductivity of from about 10.0 µSiemens/cm to about 400 µSiemens/cm; optionally said highly conductive liquid composition has a surface viscoelastic modulus of about 5.0 mN/m, a phase angle of about 25 degrees, and a conductivity of from about 50.0 µSiemens/cm to about 90.0 µSiemens/cm.

15. The method according to claim 12 wherein the aqueous liquid carrier vehicle comprises:
(i) about 95 wt % to about 100 wt % water;
(ii) about 0 wt % to about 5 wt % of a co-solvent;
(iii) about 0.1 wt % to about 2.5 wt % of an acceptable surfactant; and
(iv) about 0.1 wt % to about 2.5 wt % of an excipient;
the diameter of the aerosol particle is about 100 microns to about 350 microns; and
said highly conductive liquid composition has a surface viscoelastic modulus of from about 2.0 mN/m to about 7.5 mN/m, a phase angle of from about 10 degrees to about 50 degrees, and a conductivity of from about 10.0 µSiemens/cm to about 400 µSiemens/cm.
